(19) Europäisches Patentamt

European Patent Office

Office européen des brevets

(11) **EP 0 540 784 B1**

(12) **EUROPEAN PATENT SPECIFICATION**

(45) Date of publication and mention
of the grant of the patent:
**19.01.2000 Bulletin 2000/03**

(51) Int. Cl.[7]: **C11D 3/386**, C12N 9/42,
C11D 17/06, C11D 3/00,
C12N 15/56

(21) Application number: **91202882.6**

(22) Date of filing: **06.11.1991**

(54) **Dye transfer inhibiting compositions**

Zusammensetzungen zur Verhinderung der Farbstoffübertragung

Compositions empêchant le transfert de colorant

(84) Designated Contracting States:
**GB**

(43) Date of publication of application:
**12.05.1993 Bulletin 1993/19**

(73) Proprietor:
**THE PROCTER & GAMBLE COMPANY
Cincinnati, Ohio 45202 (US)**

(72) Inventors:
• **McCorquodale, Finlay
B-1200 Woluwe-St.-Lambert (BE)**
• **Busch, Alfred
B-2910 Londerzeel (BE)**

(74) Representative:
**Canonici, Jean-Jacques et al
Procter & Gamble European Technical Center
N.V.
Temselaan 100
1853 Strombeek-Bever (BE)**

(56) References cited:
**EP-A- 0 158 260       EP-A- 0 350 098
EP-A- 0 381 397       WO-A-89/09259
WO-A-89/09813        FR-A- 2 610 947
GB-A- 2 101 167       US-A- 4 435 307**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Xerox (UK) Business Services
2.16.7/3.6

**Description**

Field of the invention

The present invention relates to an enzymatic composition for inhibiting the transfer of dye from a dyed fabric to another fabric during washing.

Background of the invention

One of the most persistent and troublesome problems arising during modern fabric laundering operations is the tendency of some colored fabrics to release dye into the laundering solutions. The dye is then transferred onto other fabrics being washed therewith.

One way of overcoming this problem would be to bleach the fugitive dyes washed out of dyed fabrics before they have the opportunity to become attached to other articles in the wash.

Suspended or solubilized dyes can to some degree be oxidized in solution by employing known bleaching agents.

GB 2 101 167 describes a stable liquid bleaching composition containing a hydrogen peroxide precursor which is activated to yield hydrogen peroxide on dilution.

However, it is important at the same time not to bleach the dyes actually remaining on the fabrics, that is, not to cause color damage.

U,S, Patent 4,077,768 describes a process for inhibiting dye transfer by the use of an oxidizing bleaching agent together with catalytic compounds such as iron porphins.

U.S. Patent Application 421,414 describes peroxidases and oxidases utilized for the oxidation of organic or inorganic substances, including coloured substances. A dye transfer inhibiting composition comprising an enzymatic system capable of generating hydrogen peroxide and iron catalysts has been disclosed in copending EP Patent Application 91202655.6 filed October 9, 1991.

EP 424 398-A describes a detergent additive capable of exerting a bleaching effect comprising a peroxidase. The additive further comprises one or more enzymes, particularly a lipase, protease, amylase or a cellulase.

EP-A-350 098 discloses a C14CMC-method which defines a cellulase selection criteria relevant for detergent application. A minimum of 10% removal of immobilized radioactive labelled carboxymethylcellulose according to the CMC-method at $25 \times 10^{-6}$% by weight of the cellulase protein in the test solution has been found to provide high active cellulase.

A preferred group of cellulase falling under the high activity definition according to the present invention has been disclosed in copending Danish Patent Application No. 1159/90 filed May 5, 1990. There is disclosed a cellulase preparation consisting essentially of a homogeneous endoglucanase component which is immunoreactive with a monoclonal antibody raised against a partially purified = 43kD cellulase derived from Humicola insolens DM1800.

It has now been surprisingly found that the efficiency of peroxidases in terms of dye transfer inhibition, is considerably enhanced by using said high activity cellulase and more in particular the specific cellulase preparation disclosed in copending Danish Patent Application No. 1159/90. It is therefore an object of the present invention to provide dye transfer inhibiting compositions which exhibit optimum dye transfer in wash liquids by using said high activity cellulase and peroxidase.

According to one embodiment of this invention a dye transfer inhibiting composition is provided which comprises a cost-effective cellulase preparation , e.g. by employing recombinant DNA techniques.

According to another embodiment of this invention a process is also provided for laundering operations involving colored fabrics.

Summary of the Invention

The present invention provides a dye transfer inhibiting composition comprising an enzyme exhibiting peroxidase activity, a hydrogen peroxide or a hydrogen peroxide precursor or an enzymatic system capable of generating hydrogen peroxide, an additional oxidizable substrate, and a cellulase, characterized in that the cellulase provides at least 10% removal of immobilized radioactive labelled carboxymethyl cellulose according to C14CMC method at $25 \times 10^{-6}$% by weight of the laundry test solution.

According to the present invention, a preferred cellulase consists essentially of a homogeneous endoglucanase component which is immunoreactive with a monoclonal antibody raised against a partially purified = 43kD cellulase derived from Humicola insolens DM1800.

Detailed Description of the Invention

CELLULASE

The activity of enzymes and particularly the activity of cellulase enzyme has been defined for various applications by different analytical methods. These methods all attempt to provide a realistic assessment of the expected in use performance or at least a measurement correlating with the in use performance. As has been detailed in European Patent Application EP-A-350098, many of the methods, particularly these frequently used by cellulase manufacturers, are not sufficiently correlated with the in use performance of cellulase in laundry detergent compositions. This is due to the various other usage conditions for which these activity measurement methods have been developed.

The method described in EP-A-350098, has been developed to be and to have a predictive correlation for the ranking of cellulase activity in laundry detergent compositions.

The present invention therefore uses the method disclosed in EP-A-350098 to screen cellulases in order to distinguish cellulases which are useful in the present invention and those which would not provide the objectives of the present invention. The screening method, hereinafter referred to as C14CMC-Method, which has been adopted from the method disclosed in EP-A-350098, can be described as follows :

Principle :

The principle of the C14CMC-Method for screening is to measure at a defined cellulase concentration in a wash solution the removal of immobilized carboxy methyl cellulose (CMC) from a cloth substrate. The removal of CMC is measured by radio-active labelling of some of the CMC by using C14 radio-active carbon. Simple counting of the amount of radio-active C14 on the cloth substrate before and after the cellulase treatment allows the evaluation of the cellulase activity.

Sample preparation :

**CMC preparation :** The radio-active CMC stock solution is prepared according to Table I. The radio-active CMC can be obtained by methods referred to in EP-A-350098.

**Fabric substrates :** The fabric substrates are muslin cotton swatches having a size of 5 cm x 5 cm. They are inocculated with 0.35 ml of the radio-active labelled CMC stock solution in their center. The muslin cotton swatches are then airdried.

**Immobilization of CMC :** To immobilize the radio-active labelled CMC on the muslin cotton swatches, laundero-meter equipment " Linitest Original Haunau " made by Original Haunau, Germany, is used. A metal jar of the laundero-meter is filled with 400 ml of hard water (4 mmol/liter of $Ca^{++}$ ions). A maximum number of 13 swatches can be used per jar. The jar is then incubated in a heat-up cycle from 20°C to 60°C over 40 minutes in the laundero-meter equipment. After incubation the swatches are rinsed under running city water for 1 minute. They are squeezed and allowed to airdry for at least 30 minutes. According to EP-A-350098 samples of the swatches with immobilized radio-active CMC can also be measured as "blank samples" without washing.

Sample treatment :

**Laundry test solution :** The laundry test solution is prepared according to the composition of Table II. It is balanced to pH 7.5. The laundry test solution is the basis to which a cellulase test sample is added. Care should be taken to not dilute the laundry test solution by adding water to a 100% balance prior to having determined the amount of cellulase to be added. The amount of cellulase which is used in this screening test should be added to provide $25 \times 10^{-6}$ weight percent of cellulase protein in the laundry test solution (equivalent to 0.25 milligram/liter at 14.5 °C).

**Wash procedure :** The swatches thus inocculated with radio-active labelled CMC are then treated in a laundry simulation process. The laundry process is simulated in the laundero-meter type equipment," Linitest, Original Haunau", by Original Haunau, Haunau Germany. An individual swatch is put into a 20 $cm^3$ glass vial. The vial is filled with 10 ml of the laundry test solution and then sealed liquid tight. Up to 5 vials are put into each laundero-meter jar. The jar is filled with water as a heat tranfer medium for the laundering simulation. The laundering simulation is conducted as a heat-up cycle from 20°C to 60°C over 40 minutes.

After the processing of the samples the vials are submerged in cold water and subsequently each swatch is taken out of its vial, rinsed in a beaker under running soft water, squeezed and allowed to airdry for at least 30 minutes.

Measurement :

In order to measure radio-active labelled CMC removal, a scintillation counter, for example, a LKB 1210 Ultrabeta Scintillation Counter, is used. In order to obtain most accurate results, the instruction manual for optimum operation of the particular scintillation counter should be followed. For example, for the LKB 1210 Ultrabeta Scintillation Counter, the following procedure should be followed. The swatch to be measured is put into a plastic vial filled with 12 ml of scintillator liquid (e.g. scintillator 299 from Packard). The swatch is then allowed to stabilize for at least 30 minutes. The vial is then put into the LKB 1210 Ultrabeta Scintillation Counter and the respective radio-activity counts for the swatch is obtained.

In order to measure the amount of CMC removal due only to the cellulase, a measurement of a swatch which has been inocculated at the same time but has been treated in the laundry test solution without cellulase, is necessary. The activity of the cellulose is then expressed as percent of radio-active labelled CMC removal. This percentage is calculated by the following formula :

$$\% \text{ of radio-active CMC removal} = \frac{XO - XC}{XO} \times 100$$

Wherein

XO is the radioactivity scintillation count of a swatch treated with the laundry test solution without cellulose
XC is the radioactivity scintillation count of a swatch treated with the laundry test solution containing the cellulose to be evaluated

**Statistical considerations, procedure confirmation :**

In order to provide statistically sound results, standard statistical analysis should be employed. For the given example, using the LKB 1210 Ultrabeta Scintillation Counter, it has been found that a sample size of 3 swatches for each radioactivity scintillation count can be used.

In order to confirm the procedure by internal crosschecking, measurement and calculation of the "blank sample" according to EP-A-350098 are recommended. This will allow to detect and eliminate errors.

Interpretation of results :

The described screening test does provide a fast, unique and reliable method to identify cellulases which satisfy the activity criteria of the present invention versus cellulases which are not part of the present invention.

It has been found that a removal of 10% or more of the immobilized radioactive labelled CMC according to the above C14CMC-method, indicates that the respective cellulase satisfies the requirements of the invention.

It will be obvious to those skilled in the art that removal percentages above 10% indicate a higher activity for the respective cellulase. It therefore is contemplated that cellulase providing above 25% or preferably above 50% removal of radioactive labelled CMC, at the protein concentration in the laundry test solution according to the C14CMC-method, would provide indication of an even better performance of the cellulase for use in laundry detergents.

It also has been contemplated that usage of higher concentrations of cellulase for C14CMC-method, would provide higher removal percentages. However, there exists no linear proven correlation between cellulase concentration and removal percentage obtained by it.

It also has been contemplated that usage of higher concentrations of cellulase for C14CMC-method, would provide higher removal percentages.

TABLE I

| Radioactive $C_{14}$ labelled CMC stock solution (all percentages by weight of total solution) | |
|---|---|
| Total CMC* (CMC should be detergent grade CMC with a degree of substitution from about 0.47 to about 0.7) | $99.2 \times 10^{-3}$% |
| Ethanol | $14985.12 \times 10^{-3}$% |
| Deionized Water | $84915.68 \times 10^{-3}$% |
| Total : | 100% |

* Total CMC contains non-radio-active and radio-active CMC to provide a radio-activity which allows sufficiently clear readings on the scintillation counter used. For example, the radio-active CMC can have an activity of 0.7 millicurie/g and be mixed with non-radio-active CMC at a ratio of 1:6.7.

TABLE II

| Laundry test solution (all percentages by weight of total solution) | |
|---|---|
| Linear $C_{12}$ alkyl benzene sulphonic acid | 0.110% |
| Coconut alkyl sulphate (TEA salt) | 0.040% |
| $C_{12-15}$ alcohol ethoxylate (E07) | 0.100% |
| Coconut fatty acid | 0.100% |
| Oleic acid | 0.050% |
| Citric acid | 0.010% |
| Triethanolamine | 0.040% |
| Ethanol | 0.060% |
| Propanediol | 0.015% |
| Sodium hydroxide | 0.030% |
| Sodium formate | 0.010% |
| Protease | 0.006% |
| Water (2.5 mmol/liter $Ca^{++}$), pH adjustment agent (HCL or NaOH solutions) and cellulase | balance to 100% |

According to the present invention, preferred cellulases are those as described in Danish Patent Application 1159/90. For example, a cellulase preparation useful in the compositions of the invention can consist essentially of a homogeneous endoglucanase component, which is immunoreactive with an antibody raised against a highly purified 43kD cellulase derived from Humicola insolens, DSM 1800, or which is homologous to said 43kD endoglucanase.

It should be stressed that all cellulase enzymes according to the present invention have to meet the criteria of the above mentioned screening test. However, in the Danish Patent Application 1159/90 additional criteria are established allowing to identify preferred cellulase enzymes in combination with present screening test.

Cellulase preparations particulary useful in the compositions of the invention are those in which in addition to the screening test, the endoglucanase component exhibits a CMC-endoase activity of at least about 50, preferably at least about 60, in particular at least about 90 CMC-endoase units per mg of total protein. In particular, a preferred endoglucanase component exhibits a CMC-endoase activity of at least 100 CMC-endoase units per mg of total protein.

In the present context, the term "CMC-endoase activity" (cevu) refers to the endoglucanase activity of the endoglucanase component in terms of its ability to degrade cellulose to glucose, cellobiose and triose, as determined by a viscosity decrease of a solution of carboxymethyl cellulose (CMC) after incubation with the cellulase preparation of the invention, as described in detail below.

The CMC-endoase (endoglucanase) activity can be determined from the viscosity decrease of CMC, as follows : A substrate solution is prepared, containing 35 g/l CMC (Hercules 7 LFD) in 0.1 M tris buffer at pH 9.0. The enzyme sample to be analyzed is dissolved in the same buffer. 10 ml substrate solution and 0.5 ml enzyme solution are mixed and transferred to a viscosimeter (e.g. Haake VT 181, NV sensor, 181 rpm), thermostated at 40°C. Viscosity readings are taken as soon as possible after mixing and again 30 minutes later. The amount of enzyme that reduces the viscosity to one half under these conditions is defined as 1 unit of CMC-endoase activity.

SDS polyacrylamide gel electrophoresis (SDS-PAGE) and isoelectric focusing with marker proteins in a manner known to persons skilled in the art were used to determine the molecular weight and isoelectric point (pI), respectively, of the endoglucanase component in the cellulase preparation useful in the present context. In this way, the molecular weight of a specific endoglucanase component was determined to be 43kD. The isoelectric point of this endoglucanase was determined to be about 5.1.

The cellobiohydrolase activity may be defined as the activity towards cellobiose p-nitrophenyl. The activity is determined as μmole nitrophenyl released per minute at 37°C and pH 7.0. The present endoglucanase component was found to have essentially no cellobiohydrolase activity.

The endoglucanase component in the cellulase preparation herein has initially been isolated by extensive purification procedures, i.a. involving reverse phase HPLC purification of a crude H. insolens cellulase mixture according to U.S. 4,435,307. This procedure has surprisingly resulted in the isolation of a 43kD endoglucanase as a single component with unexpectedly favourable properties due to a surprisingly high endoglucanase activity.

Also, in addition to the screening test, the cellulase enzymes useful in the present compositions can further be defined as enzymes exhibiting endoglucanase activity (in the following referred to as an "endoglucanase enzyme"), which enzymes have the amino acid sequence shown in the appended Sequence Listing ID#2, or a homologue thereof exhibiting endoglucanase activity.

In the present context, the term "homologue" is intended to indicate a polypeptide encoded by DNA which hybridizes to the same probe as the DNA coding for the endoglucanase enzyme with this amino acid sequence under certain specified conditions (presoaking in 5xSSC and prehybridizing for 1 h at 40°C in a solution of 20% formamide, 5xbenhardt's solution, 50 mM sodium phosphate, pH 6.8, and 50 ug of denatured sonicated calf thymus DNA, followed by hybridization in the same solution supplemented with 100 μM ATP for 18 h at 40°C). The term is intended to include derivatives of the aforementioned sequence obtained by addition of one or more amino acid residues to either or both the C- and N-terminal of the native sequence, substitution of one or more amino acid residues at one or more sites in the native sequence, deletion of one or more amino acid residues at either or both ends of the native amino acid sequence or at one or more sites within the native sequence, or insertion of one or more amino acid residues at one or more sites in the native sequence.

The endoglucanase enzyme herein may be one producible by species of Humicola such as Humicola insolens e.g. strain DSM 1800, deposited on October 1, 1981 at the Deutsche Sammlung von Mikroorganismen, Mascheroder Weg 1B, D-3300 Braunschweig, FRG, in accordance with the provisions of the Budapest Treaty on the International Recognition of the Deposit of Microorganisms for the Purposes of Patent Procedure (the Budapest Treaty).

In still a further aspect, the cellulase enzymes useful herein can be defined, in addition to the screening test, as endoglucanase enzymes which have the amino acid sequence shown in the appended Sequence Listing ID#4, or a homologue thereof (as defined above) exhibiting endoglucanase activity. Said endoglucanase enzyme may be one producible by a species of Fusarium, such as Fusarium oxysporum, e.g. strain DSM 2672, deposited on June 6, 1983 at the Deutsche Sammlung von Mikroorganismen, Mascheroder Weg 1B, D-3300 Braunschweig, FRG, in accordance with the provisions of the Budapest Treaty.

Furthermore, it is contemplated that homologous endoglucanases may be derived from other microorganisms producing cellulolytic enzymes, e.g. species of Trichoderma, Myceliophthora, Phanerochaete, Schizophyllum, Penicillium, Aspergillus, and Geotricum.

For industrial production of the cellulase preparation herein, however, it is preferred to employ recombinant DNA techniques or other techniques involving adjustements of fermentations or mutation of the microorganisms involved to ensure overproduction of the desired enzymatic activities. Such methods and techniques are known in the art and may readily be carried out by persons skilled in the art.

The endoglucanase component may thus be one which is producible by a method comprising cultivating a host cell transformed with a recombinant DNA vector which carries a DNA sequence encoding said endoglucanase component or a precursor of said endoglucanase component as well as DNA sequences encoding functions permitting the expression of the DNA sequence encoding the endoglucanase component or precursor thereof, in a culture medium under conditions permitting the expression of the endoglucanase component or precursor thereof and recovering the endoglucanase component from the culture.

DNA constructs comprising a DNA sequence encoding an endoglucanase enzyme as described above, or a precursor form of the enzyme, include the DNA constructs having a DNA sequence as shown in the appended Sequence Listings ID#1 or ID#3, or a modification thereof. Examples of suitable mofidications of the DNA sequence are nucleotide substitutions which do not give rise to another amino acid sequence of the endoglucanase, but which correspond to the codon usage of the host organism into which the DNA construct is introduced or nucleotide substitutions which do give rise to a different amino acid sequence and therefore, possibly, a different protein structure which might give rise to an endoglucanase mutant with different properties than the native enzyme. Other examples of possible modifications are insertion of one or more nucleotides at either end of the sequence, or deletion of one or more nucleotides at either end or within the sequence.

DNA constructs encoding endoglucanase enzymes useful herein may be prepared synthetically by established standard methods, e.g. the phosphoamidite method described by S.L. Beaucage and M.H. Caruthers, Tetrahedron Letters 22, 1981, pp. 1859-1869, or the method described by Matthes et al., EMBO Journal 3, 1984, pp. 801-805. According to the phosphoamidite method, oligonucleotides are synthesized, e.g. in an automatic DNA synthesizer, purified, annealed, ligated and cloned in suitable vectors.

A DNA construct encoding the endoglucanase enzyme or a precursor thereof may, for instance, be isolated by establishing a cDNA or genomic library of a cellulase-producing microorganism, such as Humicola insolens, DSM 1800, and screening for positive clones by conventional procedures such as by hybridization using oligonucleotide probes synthesized on the basis of the full or partial amino acid sequence of the endoglucanase in accordance with standard techniques (cf. Sambrook et al., Molecular Cloning: A Laboratory Manual, 2nd. Ed. Cold Spring Harbor, 1989), or by selecting for clones expressing the appropriate enzyme activity (i.e. CMC-endoase activity as defined above), or by selecting for clones producing a protein which is reactive with an antibody against a native cellulase (endoglucanase).

Finally, the DNA construct may be of mixed synthetic and genomic, mixed synthetic and cDNA or mixed genomic and cDNA origin prepared by ligating fragments of synthetic, genomic or cDNA origin (as appropriate), the fragments corresponding to various parts of the entire DNA construct, in accordance with standard techniques. The DNA construct may also be prepared by polymerase chain reaction using specific primers, for instance as described in US 4,683,202 or R.K. Saiki et al., Science 239, 1988, pp. 487-491.

Recombinant expression vectors into which the above DNA constructs are inserted include any vector which may conveniently be subjected to recombinant DNA procedures, and the choice of vector will often depend on the host cell into which it is to be introduced. Thus, the vector may be an autonomously replicating vector, i.e. a vector which exists as an extrachromosomal entity, the replication of which is independent of chromosomal replication, e.g. a plasmid. Alternatively, the vector may be one which, when introduced into a host cell, is integrated into the host cell genome and replicated together with the chromosome(s) into wich it has been integrated.

In the vector, the DNA sequence encoding the endoglucanase should be operably connected to a suitable promoter and terminator sequence. The promoter may be any DNA sequence which shows transcriptional activity in the host cell of choice and may be derived from genes encoding proteins either homologous or heterologous to the host cell. The procedures used to ligate the DNA sequences coding for the endoglucanase, the promoter and the terminator, respectively, and to insert them into suitable vectors are well known to persons skilled in the art (cf., for instance, Sambrook et al., op.cit.).

Host cells which are transformed with the above DNA constructs or the above expression vectors may be for instance belong to a species of Aspergillus, most preferably Aspergillys oryzae or Aspergillus niger. Fungal cells may be transformed by a process involving protoplast formation and transformation of the protoplasts followed by regeneration of the cell wall in a manner known per se. The use of Aspergillus as a host microorganism is described in EP 238 023 (of Novo Industri A/S), the contents of which are hereby incorporated by reference. The host cell may also be a yeast cell, e.g. a strain of Saccharomyces cerevisiae.

Alternatively, the host organism may be a bacterium, in particular strains of Streptomyces and Bacillus, and E. coli. The transformation of bacterial cells may be performed according to conventional methods, e.g. as described in Sambrook et al., Molecular Cloning: A Laboratory Manual, Cold Spring Harbor, 1989.

The screening of appropriate DNA sequences and construction of vectors may also be carried out by standard procedures, cf. Sambrook et al., op.cot.

The medium used to cultivate the transformed host cells may be any conventional medium suitable for growing the host cells in question. The expressed endoglucanase may conveniently be secreted into the culture medium and may be recovered therefrom by well-known procedures including separating the cells from the medium by centrifugation or filtration, precipitating proteinaceous components of the medium by means of a salt such as ammonium sulphate, followed by chromatographic procedures such as ion exchange chromatography, affinity chromatography, or the like.

By employing recombinant DNA techniques as indicated above, techniques of protein purification, techniques of fermentation and mutation or other techniques which are well known in the art, it is possible to provide endoglucanases of a high purity.

The level in the present composition of cellulase described above should be such that the amount of enzyme protein to be delivered in the wash solution is from 0.005 to 40 mg/liter of wash solution, preferably 0.01 to 10 mg/liter of wash solution.

PEROXIDASES

The peroxidases which may be employed for the present purpose may be isolated from and are producible by plants (e.g. horseradish peroxidase) or micoorganisms such as fungi or bacteria. Some preferred fungi include strains belonging to the subdivision Deuteromycotina, class Hyphomycetes, e.g. Fusarium, Humicola, Tricoderma, Myrothecium, Verticillum, Arthromyces, Caldariomyces, Ulocladium, Embellisia, Cladosporium or Dreschlera, in particular Fusarium oxysporum (DSM 2672), Humicola insolens, Trichoderma resii, Myrothecium verrucana (IFO 6113), Verticilluum alboatrum, Verticillum dahlie, Arthromyces ramosus (FERM P-7754), Caldariomyces fumago, Ulocladium chartarum, Embellisia allior Dreschlera halodes.

Other preferred fungi inlcude strains belonging to the subdivision Basidiomycotina, class Basidiomycetes, e.g. Coprinus, Phanerochaete, Coriolus or Trametes, in particular Coprinus cinereus f. microsporus (IFO 8371), Coprinus macrorhizus, Phanerochaete chrysosporium (e.g. NA-12) or Coriolus versicolor (e.g. PR4 28-A).

Further preferred fungi include strains belonging to the subdivision Zygomycotina, class Mycoraceae, e.g. Rhizopus or Mucor, in particular Mucor hiemalis.

Some preferred bacteria include strains of the order Actinomycetales, e.g. Streptomyces spheroides (ATTC 23965), Streptomyces thermoviolaceus (IFO 12382) or Streptoverticillum verticillium ssp. verticillium.

Other preferred bacteria inlude Bacillus pumillus (ATCC 12905), Bacillus stearothermophilus, Rhododbacter sphaeroides, Rhodomonas palustri, Streptococcus lactis, Pseudomonas purrocinia (ATCC 15958) or Pseudomonas fluorescens (NRRL B-11).

Other potential sources of useful peroxidases are listed in B.C. Saunders et al., op. cit., pp. 41-43.

Methods of producing enzymes to be used according to the invention are described in the art, cf. for example FEBS Letters 1625, 173(1), Applied and Environmental Microbiology, Feb. 1985, pp. 273-278, Applied Microbiol. Biotechnol. 26, 1987, pp. 158-163, Biotechnology Letters 9(5), 1987, pp. 357-360, Nature 326, 2 April 1987, FEBS Letters 4270, 209(2), p. 321, EP 179 486, EP 200 565, GB 2 167 421, EP 171 074, and Agric. Biol. Chem. 50(1), 1986, p. 247.

Particularly preferred peroxidases are those which are active at the typical pH of washing liquors, i.e. at a pH of 6.5-10.5, preferably 6.5-9.5, and most preferably 7.5-9.5. Such enzymes may be isolated by screening for the relevant enzyme production by alkalophilic microorganisms, e.g. using the ABTS assay described in R.E. Childs and W.G. Bardsley, Biochem. J.145, 1975, pp. 93-103.

Other preferred peroxidases are those which exhibit a good thermostability as well as a good stability towards commonly used detergent components such as non-ionic, cationic, or anionic surfactants, detergent builders, phosphate etc.

Another group of useful peroxidases are haloperoxidases, such as chloro- and bromoperoxidases.

The peroxidase-enzyme may futhermore be one which is producible by a method comprising cultivating a host cell transformed with a recombinant DNA vector which carries a DNA sequence encoding said enzyme as well as DNA sequences encoding functions permitting the expression of the DNA sequence encoding the enzyme, in a culture medium under conditions permitting the expression of the enzyme and recovering the enzyme from the culture.

A DNA fragment encoding the enzyme may, for instance, be isolated by establishing a cDNA or genomic library of a microorganism producing the enzyme of interest, such as one of the organisms mentioned above, and screening for positive clones by conventional procedures such as by hybridization to oligonucleotide probes synthesized on the basis of the full or partial amino acid sequence of the enzyme, or by selecting for clones expressing the appropriate enzyme activity , or by selecting for clones producing a protein which is reactive with an antibody against the native enzyme.

Once selected, the DNA sequence may be inserted into a suitable replicable expression vector comprising appropriate promotor, operator and terminator sequences permitting the enzyme to be expressed in a particular host organism, as well as an origin of replication, enabling the vector to replicate in the host organism in question.

The resulting expression vector may then be transformed into a suitable host cell, such as a fungal cell, preferred examples of which are a species of Aspergillus, most preferably Aspergillus oryzae or Aspergillus niger. Fungal cells may be transformed by a process involving protoplast formation and transformation of the protoplasts followed by regeneration of the cell wall in a manner known per se. The use of Aspergillus as a host micoorganism is described in EP 238,023 (of Novo Industri A/S), the contents of which are hereby incorporated by reference.

Alternatively, the host organisms may be a bacterium, in particular strains of Streptomyces and Bacillus, or E. coli. The transformation of bacterial cells may be performed according to conventional methods, e.g. as described in T. Maniatis et al., Molecular Cloning : A Laboratory Manual, Cold Spring Harbor, 1982.

The screening of appropriate DNA sequences and construction of vectors may also be carried out by standard procedures, cf. T. Maniatis et al., op. cit.

The medium used to cultivate the transformed host cells may be any conventional medium suitable for growing the host cells in question. The expressed enzyme may conveniently be secreted into the culture medium and may be recovered therefrom by well-known procedures including separating the cells from the medium by centrifugation or filtration, precipitating proteinaceous components of the medium by means of a salt such as ammonium sulphate, followed by chromatographic procedures such as ion exchange chromatography, affinity chromatography, or the like.

The screening of appropriate DNA sequences and construction of vectors may also be carried out by standard procedures, cf. T. Maniatis et al., op. cit.

The medium used to cultivate the transformed host cells may be any conventional medium suitable for growing the host cells in question. The expressed enzyme may conveniently be secreted into the culture medium and may be recovered therefrom by well-known procedures including separating the cells from the medium by centrifugation or filtration, precipitating proteinaceous components of the medium by means of a salt such as ammonium sulphate, followed by chromatographic procedures such as ion exchange chromatography, affinity chromatography, or the like.

At the beginning or during the process, $H_2O_2$ may be added, e.g. in an amount of 0.001-5 mM, particularly 0.01-1 mM. When using Coprinus peroxidase, 0.01-0.25 mM $H_2O_2$ is preferred, and with B. pumilus peroxidase 0.1-1 mM $H_2O_2$.

The hydrogen peroxide may be added as hydrogen peroxide or a precursor thereof, preferably a perborate or percarbonate. The level of hydrogen peroxide precursor that can be used is dependent on the specific properties of the peroxidase chosen, e.g. Coprinus peroxidase should be applied in a detergent composition which contains less than 5% perborate.

In the process of this invention, it may be desirable to utilize an enzymatic process for hydrogen peroxide formation. Thus, the process according to the invention may additionally comprise adding an enzymatic system (i.e. an enzyme and a substrate therefor) which is capable of generating hydrogen peroxide at the beginning or during the washing and/or rinsing process.

One such category of hydrogen peroxide generating systems comprises enzymes which are able to convert molecular oxygen and an organic or inorganic substrate into hydrogen peroxide and the oxidized substrate respectively. These enzymes produce only low levels of hydrogen peroxide, but they may be employed to great advantage in the process of the invention as the presence of peroxidase ensures an efficient utilization of the hydrogen peroxide produced.

Preferred hydrogen peroxide-generating enzymes are those which act on cheap and readily available substrates which may conveniently be included into detergent compositions. An example of such a substrate is glucose which may be utilized for hydrogen peroxide production by means of glucose oxidase. Suitable oxidases include those which act on aromatic compounds such as phenols and related substances, e.g. catechol oxidases, laccase. Other suitable oxidases are urate oxidase, galactose oxidase, alcohol oxidases, amine oxidases, amino acid oxidase, amyloglucosidase, and cholesterol oxidase.

The preferred enzymatic systems are alcohol and aldehyde oxidases.

The more preferred systems for granular detergent application would have solid alcohols, e.g. glucose whose oxidation is catalysed by glucose oxidase to glucoronic acid with the formation of hydrogen peroxide.

The more preferred systems for liquid detergent application would involve liquid alcohols which could also act as, for example, solvents. An example is ethanol/ethanol oxidase.

The quantity of oxidase to be employed in compositions according to the invention should be at least sufficient to provide a constant generation of 0.01 to 10 ppm AvO per minute in the wash. For example, with the glucose oxidase, this can be achieved at room temperature and at pH 6 to 11, preferentially 7 to 9 with 50-5000 U/l glucose oxidase, 0.005 to 0.5 % glucose under constant aeration.

The addition of another oxidisable substrate for the peroxidase at the beginning or during the washing and/or rinsing process may enhance the dye transfer inhibitory effect of the peroxidase employed. This is thought to be ascribable to the formation of short-lived radicals or other oxidised states of this substrate which participate in the bleaching or other modification of the coloured substance. Examples of such oxidisable substrates are metal ions, e.g. $Mn^{++}$, halide ions, e.g. chloride or bromide ions, or organic compounds such as phenols, e.g. p-hydroxycinnamic acid or 2,4-dichlorophenol. Other examples of phenolic compounds which may be used for the present purpose are those given in M. Kato and S. Shimizu, Plant Cell Physiol. 26(7), 1985, pp. 1291-1301 (cf. Table 1 in particular) or B.C. Saunders et al., op. cit., p. 141 ff. The amount of oxidisable substrate to be added is suitably between about 1 μM and 1 mM.

In the process of the invention, the peroxidase will typically be added as a component of a detergent composition and may be added in an amount of 0.01 - 100 mg enzyme per liter of wash liquid. As such, it may be included in the detergent composition in the form of a non-dusting granulate, a liquid, in particular a stabilized liquid, or a protected enzyme. Non-dusting granulates may be produced, e.g. as disclosed in US 4,106,991 and 4,661,452 (both to Novo Industri A/S) and may optionally be coated by methods known in the art. Liquid enzyme preparations may, for instance, be stabilized by adding a polyol such as propylene glycol, a sugar or sugar alcohol, lactic acid or boric acid according to established methods. Other enzyme stabilizers are well known in the art. Protected enzymes may be prepared according to the method disclosed in EP 238,216. The detergent composition may also comprise one or more substrates for the peroxidase. Usually, the pH of a solution of the detergent composition of the invention will be preferably from 7-12, especially from 7.5 to 9.5. The wash pH is dependent on the peroxidase chosen, e.g. Coprinus peroxidase should be applied in a wash pH below 9.5.

Detergent adjuncts

The composition of the present can contain the usual components of such detergent compositions in the usual amounts. Thus, organic surfactants anionic, nonionic, ampholytic, or zwitterionic or less usually cationic and mixtures thereof, may be present. Suitable surfactants are well known in the art and an extensive list of such compounds is given in US Pat. No. 3,717,630 and in US patent application Ser. No. 589,116.

Detergent compositions useful in the present invention contain from 1 to 95% , preferable from 5 to 40% of a nonionic, anionic, zwitterionic, or mixtures thereof. Detergency builders, whether inorganic or organic, phosphatic or not, water-soluble or insoluble, and other water-soluble salts may be present, and salts of this sort may be employed whether organic detergents are present or not. A description of suitable builders is given in US Pat. No. 3,936,537 and in US patent application Ser. No. 589,116. Detergent builders are present from 0 to 50%, preferably from 5 to 40%.

Other components used in detergent compositions may be employed, such as suds boosting or depressing agents, enzymes and stabilizers or activators, soil-suspending agents soil-release agents, optical brighteners, abrasives, bactericides, tarnish inhibitors, coloring agents, and perfumes.

These components, particularly the enzymes, optical brighteners, coloring agents, and perfumes, should preferably be chosen such that they are compatible with the bleach component of the composition.

The detergent compositions according to the invention can be in liquid, paste or granular forms. The enzyme may be formulated in any convenient form, e.g. as a powder or liquid. The enzyme may be stabilized in a liquid by inclusion of enzyme stabilizers. Liquid deterents may further include stabilized hydrogen peroxide precursors. Granular compositions according to the present invention can also be in "compact form", i.e. they may have a relatively higher density than conventional granular detergents, i.e. from 550 to 950 g/l; in such case, the granular detergent compositions according to the present invention will contain a lower amount of "inorganic filler salt", compared to conventional granular detergents; typical filler salts are alkaline earth metal salts of sulphates and chlorides, typically sodium sulphate; "compact" detergents typically comprise not more than 10% filler salt.

The present invention also relates to a process for inhibiting dye transfer from one fabric to another of solubilized and suspended dyes encountered during fabric laundering operations involving colored fabrics.

The process comprises contacting fabrics with a laundering solution as hereinbefore described.

The process of the invention is conveniently carried out in the course of the washing process. The washing process is preferably carried out at 5 °C to 75 °C, especially 20°C to 60°C. The pH of the treatment solution is preferably from 7 to 12, especially from 7 to 9.5.

The process and compositions of the invention can also be used as additive during laundry operations.

The following examples illustrate the present invention and the unexpected superior colour care benefits obtained therefrom.

EXAMPLE I

Criticality of the cellulase performance parameter of claim 1

The following test was conducted :

**Test conditions :**

    Washing temperature : 60°C (heat up cycle)
    Washing time : 40 min.
    pH = 7.5
    Water hardness : 4 mmol/L
    Detergent concentration : 1%
    Detergent composition : crf. EPA 350 098 ex. 1
    Cellulases :

        1) Celluzyme[R] supplied by Novo Nordisk
        = reference
        2) 43kD endoglucanase
        = cellulase according to the invention

| Test Results : | |
|---|---|
| | % C14-CMC Removal by Cellulase |
| Detergent without cellulase (= reference) | 0 |
| Detergent + Celluzyme$^R$ | |
| 1.5 mg protein/L (150 x $10^{-6}$%) | 12.7 |
| 3.0 mg protein/L (300 x $10^{-6}$%) | 17.7 |
| 4.5 mg protein/L (450 x $10^{-6}$%) | 21.5 |
| Detergent + 43kD endoglucanase | |
| 0.3 mg protein/L (30 x $10^{-6}$%) | 20.3 |

**Discussion of the results :**

The above data clearly demonstrate the criticality of the claimed parameter for the cellulases of the invention over the commercially available Celluzyme.

EXAMPLE II

Two sets of each four types of detergent compositions are prepared, all based on a compact granular.

Such a compact granular detergent composition typically contains the following ingredients :

| | |
|---|---|
| Linear alkyl benzene sulphonate (LAS) | 11% |
| Alkyl suphate | 5% |
| Nonionic | 6% |
| Trisodium citrate | 15% |
| Zeolite | 32% |
| Citric acid | 6% |
| Polymer | 4% |
| Chelant | 0.2% |
| Sodium sulphate | 5% |
| Sodium silicate | 2% |
| Perborate | 0.5% |
| Phenol sulphonate | 0.1% |

The above detergent composition was supplemented as indicated in below :

```
SET 1 : With 43kD endoglucanase
```

A) without 43kD endoglucanase and peroxidase = reference

B) with 43 kD endoglucanase

C) with peroxide

D) with 43kD endoglucanase and peroxidase

```
SET 2 : With Celluzyme^R
```

A) without celluzyme$^R$ and peroxidase

B) with celluzyme$^R$

C) with peroxidase

D) with celluzyme$^R$ and peroxidase

The first type of each set of detergent compositions does not contain any cellulase and peroxidase (reference composition : A ).

In the first set of detergent compositions the 43kD endoglucanase is added at a level of 2 mg enzyme protein/liter of the wash solution (55 cevu/liter).

In the second set of the detergent compositions the celluzyme$^R$ is added at a level of 76 mg enzyme protein/liter of the wash solution (55 cevu/liter).

**Test conditions :**

- Test in Miele washing machine
- Cotton program, low water level (181), short cycle
- 4 cycles
- Temperature 40°C
- Composition of the wash-load

    1) 1 kg clean load :

- 150 g cotton (terry)
- 150 g knitted cotton (underwear)
- 200 g woven cotton
- 300 g PE/cotton
- 200 g PE

    2) For whiteness grading : 3 white, soiled, items (4 replicates each).

    3) 10 by 5 cm acid red 151 dye on nylon : To create a low dye transfer level.

    4) Prepared stains to provide a source of typical laundry soil.

- Hard water (15 grs/glln).
- Detergent concentration = 0.6%.

**Test procedure :**

The design of the test is such as to compare whiteness of the textile items, laundered cumulatively 4 times between the compositions to be tested and the reference composition. Three soiled items were used for this test. For each treatment of an item four replicates were used.

The items to be examined are displayed on a flat, neutral colored grading surface parallel to the light source. As a light source a fluorescent light is used : 27 Philips "cold" color TL 40/57 producing 1080 WATTS of light designed to match with regular daylight (D65). The color T° is of 7400°K, the color reflectance is excellent (94) and the light output is 46 LM/W.

Differences are recorded in panel score units (psu), positive being performancewise better than the reference treatment.

Grading scale (PSU grading)

0 = Equal
1 = I think this one is better
2 = I know this one is a little better
3 = This one is a lot better
4 = This one is a whole lot better

The PSU grading data are statistical recount, an average of the 4 replicates is made, LSD (least significant difference) is mentioned in table I and II.

TABLE I

| Test results : peroxidase / celluzyme[R] | | | | |
|---|---|---|---|---|
| | Bvs.A | Cvs.A | Dvs.A | LSD |
| 2 cycles AV | 0.28 | 1.67s | 1.80s | 0.61 |
| 3 cycles AV | 0.22 | 2.02s | 2.26s | 0.50 |
| 4 cycles AV | 0.68 | 2.48s | 2.66s | 0.76 |

TABLE II

| Test results : peroxidase / •43kD endoglucanase | | | | |
|---|---|---|---|---|
| | Bvs.A | Cvs.A | Dvs.A | LSD |
| 2 cycles AV | -0.10 | 1.38s | 1.97s | 0.40 |
| 3 cycles AV | -0.30 | 2.25s | 2.51s | 0.46 |
| 4 cycles AV | 0.07 | 2.44s | 2.97s | 0.29 |

Conclusion :

The above results clearly show that the peroxidase/43kD combination of the present invention gives a statistically significant better performance than the sum of the individual actions of both ingredients.

EXAMPLE III to VIII

The following compositions are made.

| a) Compact granular detergent : examples II to IV. | | |
|---|---|---|
| EXAMPLES | III | IV |
| Linear alkyl benzene sulphonate | 11.4 | 10.70 |
| Tallow alkyl sulphate | 1.80 | 2.40 |

**EP 0 540 784 B1**

(continued)

| a) Compact granular detergent : examples II to IV. | | |
|---|---|---|
| EXAMPLES | III | IV |
| $C_{45}$ alkyl sulphate | 3.00 | 3.10 |
| $C_{45}$ alcohol 7 times ethoxylated | 4.00 | 4.00 |
| Tallow alcohol 11 times ethoxylated | 1.80 | 1.80 |
| Dispersant | 0.07 | 0.1 |
| Silicone fluid | 0.80 | 0.80 |
| Trisodium citrate | 14.00 | 15.00 |
| Citric acid | 3.00 | 2.50 |
| Zeolite | 32.50 | 32.10 |
| Maleic acid acrylic acid copolymer | 5.00 | 5.00 |
| DETMPA | 1.00 | 0.20 |
| Cellulase 43kD endoglucanase | 0.03 | 0.025 |
| Alkalase | 0.60 | 0.60 |
| Lipase | 0.36 | 0.40 |
| Amylase | 0.30 | 0.30 |
| Sodium silicate | 2.00 | 2.50 |
| Sodium sulphate | 3.50 | 5.20 |
| PVP | 0.30 | 0.50 |
| Perborate | 0.5 | 1 |
| Phenol sulphonate | 0.1 | 0.2 |
| Peroxidase | 0.1 | 0.1 |
| Minors | up to 100 | |

| b) conventional granular detergent : examples V and VI | | |
|---|---|---|
| EXAMPLES | V | IV |
| Sodium linear $C_{12}$ alkyl benzene sulfonate | 6.5 | 8.0 |
| Sodium sulfate | 15.0 | 18.0 |
| Zeolite A | 26.0 | 22.0 |
| Sodium nitrilotriacetate | 5.0 | 5.0 |
| Cellulase 43kD endoglucanase | 0.02 | 0.03 |
| PVP | 0.5 | 0.7 |
| TAED | 3.0 | 3.0 |
| Boric acid | 4.0 | - |
| Perborate | 0.5 | 1 |
| Phenol sulphonate | 0.1 | 0.2 |

**14**

(continued)

| b) conventional granular detergent : examples V and VI | | |
|---|---|---|
| EXAMPLES | V | IV |
| Peroxidase | 0.1 | 0.2 |
| Minors | up to 100 | |

| c) liquid detergent : examples VII and VIII | | |
|---|---|---|
| EXAMPLES | VII | VIII |
| $C_{12-14}$ alkenyl succinic acid | 3.0 | 8.0 |
| Citric acid monohydrate | 10.0 | 15.0 |
| Sodium $C_{12-15}$ alkyl sulphate | 8.0 | 8.0 |
| Sodium sulfate of C12-15 alcohol 2 times ethoxylated | - | 3.0 |
| $C_{12-15}$ alcohol 7 times ethoxylated | - | 8.0 |
| $C_{12-15}$ alcohol 5 times ethoxylated | 8.0 | - |
| Diethylene triamine penta (methylene phosphonic acid) | 0.2 | - |
| Oleic acid | 1.8 | - |
| Ethanol | 4.0 | 4.0 |
| Propanediol | 2.0 | 2.0 |
| Protease | 0.2 | 0.2 |
| Cellulase 43kD endoglucanase | 0.2 | 0.05 |
| PVP | 1.0 | 2.0 |
| Suds suppressor | 0.15 | 0.15 |
| NaOH | up to | pH 7.5 |
| Perborate | 0.5 | 1 |
| Phenol sulphonate | 0.1 | 0.2 |
| Peroxidase | 0.4 | 0.1 |
| Waters and minors | up to 100 parts | |

15

SEQUENCE DESCRIPTION: SEQ ID NO:1:

```
GGATCCAAG ATG CGT TCC TCC CCC CTC CTC CCG TCC GCC GTT GTG GCC        48
          Met Arg Ser Ser Pro Leu Leu Pro Ser Ala Val Val Ala
          -21 -20                -15                  -10

GCC CTG CCG GTG TTG GCC CTT GCC GCT GAT GGC AGG TCC ACC CGC TAC      96
Ala Leu Pro Val Leu Ala Leu Ala Ala Asp Gly Arg Ser Thr Arg Tyr
            -5                  1                 5

TGG GAC TGC TGC AAG CCT TCG TGC GGC TGG GCC AAG AAG GCT CCC GTG     144
Trp Asp Cys Cys Lys Pro Ser Cys Gly Trp Ala Lys Lys Ala Pro Val
        10              15              20

AAC CAG CCT GTC TTT TCC TGC AAC GCC AAC TTC CAG CGT ATC ACG GAC     192
Asn Gln Pro Val Phe Ser Cys Asn Ala Asn Phe Gln Arg Ile Thr Asp
25              30              35                      40

TTC GAC GCC AAG TCC GGC TGC GAG CCG GGC GGT GTC GCC TAC TCG TGC     240
Phe Asp Ala Lys Ser Gly Cys Glu Pro Gly Gly Val Ala Tyr Ser Cys
            45                  50                  55

GCC GAC CAG ACC CCA TGG GCT GTG AAC GAC GAC TTC GCG CTC GGT TTT     288
Ala Asp Gln Thr Pro Trp Ala Val Asn Asp Asp Phe Ala Leu Gly Phe
            60              65                  70

GCT GCC ACC TCT ATT GCC GGC AGC AAT GAG GCG GGC TGG TGC TGC GCC     336
Ala Ala Thr Ser Ile Ala Gly Ser Asn Glu Ala Gly Trp Cys Cys Ala
        75              80                  85

TGC TAC GAG CTC ACC TTC ACA TCC GGT CCT GTT GCT GGC AAG AAG ATG     384
Cys Tyr Glu Leu Thr Phe Thr Ser Gly Pro Val Ala Gly Lys Lys Met
        90              95              100

GTC GTC CAG TCC ACC AGC ACT GGC GGT GAT CTT GGC AGC AAC CAC TTC     432
Val Val Gln Ser Thr Ser Thr Gly Gly Asp Leu Gly Ser Asn His Phe
105             110             115                 120

GAT CTC AAC ATC CCC GGC GGC GGC GTC GGC ATC TTC GAC GGA TGC ACT     480
Asp Leu Asn Ile Pro Gly Gly Gly Val Gly Ile Phe Asp Gly Cys Thr
                125             130                 135
```

```
CCC CAG TTC GGC GGT CTG CCC GGC CAG CGC TAC GGC GGC ATC TCG TCC          528
Pro Gln Phe Gly Gly Leu Pro Gly Gln Arg Tyr Gly Gly Ile Ser Ser
            140                 145                 150

CGC AAC GAG TGC GAT CGG TTC CCC GAC GCC CTC AAG CCC GGC TGC TAC          576
Arg Asn Glu Cys Asp Arg Phe Pro Asp Ala Leu Lys Pro Gly Cys Tyr
            155                 160                 165

TGG CGC TTC GAC TGG TTC AAG AAC GCC GAC AAT CCG AGC TTC AGC TTC          624
Trp Arg Phe Asp Trp Phe Lys Asn Ala Asp Asn Pro Ser Phe Ser Phe
            170                 175                 180

CGT CAG GTC CAG TGC CCA GCC GAG CTC GTC GCT CGC ACC GGA TGC CGC          672
Arg Gln Val Gln Cys Pro Ala Glu Leu Val Ala Arg Thr Gly Cys Arg
185                 190                 195                 200

CGC AAC GAC GAC GGC AAC TTC CCT GCC GTC CAG ATC CCC TCC AGC AGC          720
Arg Asn Asp Asp Gly Asn Phe Pro Ala Val Gln Ile Pro Ser Ser Ser
            205                 210                 215

ACC AGC TCT CCG GTC AAC CAG CCT ACC AGC ACC AGC ACC ACG TCC ACC          768
Thr Ser Ser Pro Val Asn Gln Pro Thr Ser Thr Ser Thr Thr Ser Thr
            220                 225                 230

TCC ACC ACC TCG AGC CCG CCA GTC CAG CCT ACG ACT CCC AGC GGC TGC          816
Ser Thr Thr Ser Ser Pro Pro Val Gln Pro Thr Thr Pro Ser Gly Cys
            235                 240                 245

ACT GCT GAG AGG TGG GCT CAG TGC GGC GGC AAT GGC TGG AGC GGC TGC          864
Thr Ala Glu Arg Trp Ala Gln Cys Gly Gly Asn Gly Trp Ser Gly Cys
            250                 255                 260

ACC ACC TGC GTC GCT GGC AGC ACT TGC ACG AAG ATT AAT GAC TGG TAC          912
Thr Thr Cys Val Ala Gly Ser Thr Cys Thr Lys Ile Asn Asp Trp Tyr
265                 270                 275                 280

CAT CAG TGC CTG TAGACGCAGG GCAGCTTGAG GGCCTTACTG GTGGCCGCAA          964
His Gln Cys Leu
            285

CGAAATGACA CTCCCAATCA CTGTATTAGT TCTTGTACAT AATTTCGTCA TCCCTCCAGG         1024

GATTGTCACA TAAATGCAAT GAGGAACAAT GAGTAC                                  1060
```

## SEQUENCE DESCRIPTION: SEQ ID NO:2:

```
Met Arg Ser Ser Pro Leu Leu Pro Ser Ala Val Val Ala Ala Leu Pro
-21 -20              -15              -10

Val Leu Ala Leu Ala Ala Asp Gly Arg Ser Thr Arg Tyr Trp Asp Cys
-5               1              5                   10

Cys Lys Pro Ser Cys Gly Trp Ala Lys Lys Ala Pro Val Asn Gln Pro
            15              20              25

Val Phe Ser Cys Asn Ala Asn Phe Gln Arg Ile Thr Asp Phe Asp Ala
        30              35              40

Lys Ser Gly Cys Glu Pro Gly Gly Val Ala Tyr Ser Cys Ala Asp Gln
    45              50              55

Thr Pro Trp Ala Val Asn Asp Asp Phe Ala Leu Gly Phe Ala Ala Thr
60              65              70              75

Ser Ile Ala Gly Ser Asn Glu Ala Gly Trp Cys Cys Ala Cys Tyr Glu
            80              85              90

Leu Thr Phe Thr Ser Gly Pro Val Ala Gly Lys Lys Met Val Val Gln
        95              100             105

Ser Thr Ser Thr Gly Gly Asp Leu Gly Ser Asn His Phe Asp Leu Asn
    110             115             120

Ile Pro Gly Gly Gly Val Gly Ile Phe Asp Gly Cys Thr Pro Gln Phe
    125             130             135

Gly Gly Leu Pro Gly Gln Arg Tyr Gly Gly Ile Ser Ser Arg Asn Glu
140             145             150             155

Cys Asp Arg Phe Pro Asp Ala Leu Lys Pro Gly Cys Tyr Trp Arg Phe
            160             165             170

Asp Trp Phe Lys Asn Ala Asp Asn Pro Ser Phe Ser Phe Arg Gln Val
            175             180             185

Gln Cys Pro Ala Glu Leu Val Ala Arg Thr Gly Cys Arg Arg Asn Asp
    190             195             200

Asp Gly Asn Phe Pro Ala Val Gln Ile Pro Ser Ser Ser Thr Ser Ser
    205             210             215
```

```
Pro Val Asn Gln Pro Thr Ser Thr Ser Thr Thr Ser Thr Ser Thr Thr
220             225             230             235

Ser Ser Pro Pro Val Gln Pro Thr Thr Pro Ser Gly Cys Thr Ala Glu
            240             245             250

Arg Trp Ala Gln Cys Gly Gly Asn Gly Trp Ser Gly Cys Thr Thr Cys
            255             260             265

Val Ala Gly Ser Thr Cys Thr Lys Ile Asn Asp Trp Tyr His Gln Cys
        270             275             280

Leu
```

SEQUENCE DESCRIPTION: SEQ ID NO:3:

```
GAATTCGCGG CCGCTCATTC ACTTCATTCA TTCTTTAGAA TTACATACAC TCTCTTTCAA        60

AACAGTCACT CTTTAAACAA AACAACTTTT GCAACA ATG CGA TCT TAC ACT CTT       114
                                        Met Arg Ser Tyr Thr Leu
                                         1               5

CTC GCC CTG GCC GGC CCT CTC GCC GTG AGT GCT GCT TCT GGA AGC GGT       162
Leu Ala Leu Ala Gly Pro Leu Ala Val Ser Ala Ala Ser Gly Ser Gly
            10              15              20

CAC TCT ACT CGA TAC TGG GAT TGC TGC AAG CCT TCT TGC TCT TGG AGC       210
His Ser Thr Arg Tyr Trp Asp Cys Cys Lys Pro Ser Cys Ser Trp Ser
        25              30              35

GGA AAG GCT GCT GTC AAC GCC CCT GCT TTA ACT TGT GAT AAG AAC GAC       258
Gly Lys Ala Ala Val Asn Ala Pro Ala Leu Thr Cys Asp Lys Asn Asp
        40              45              50

AAC CCC ATT TCC AAC ACC AAT GCT GTC AAC GGT TGT GAG GGT GGT GGT       306
Asn Pro Ile Ser Asn Thr Asn Ala Val Asn Gly Cys Glu Gly Gly Gly
 55              60              65              70

TCT GCT TAT GCT TGC ACC AAC TAC TCT CCC TGG GCT GTC AAC GAT GAG       354
Ser Ala Tyr Ala Cys Thr Asn Tyr Ser Pro Trp Ala Val Asn Asp Glu
            75              80              85

CTT GCC TAC GGT TTC GCT GCT ACC AAG ATC TCC GGT GGC TCC GAG GCC       402
Leu Ala Tyr Gly Phe Ala Ala Thr Lys Ile Ser Gly Gly Ser Glu Ala
            90              95              100
```

```
AGC TGG TGC TGT GCT TGC TAT GCT TTG ACC TTC ACC ACT GGC CCC GTC          450
Ser Trp Cys Cys Ala Cys Tyr Ala Leu Thr Phe Thr Thr Gly Pro Val
        105                 110                 115

AAG GGC AAG AAG ATG ATC GTC CAG TCC ACC AAC ACT GGA GGT GAT CTC          498
Lys Gly Lys Lys Met Ile Val Gln Ser Thr Asn Thr Gly Gly Asp Leu
    120                 125                 130

GGC GAC AAC CAC TTC GAT CTC ATG ATG CCC GGC GGT GGT GTC GGT ATC          546
Gly Asp Asn His Phe Asp Leu Met Met Pro Gly Gly Gly Val Gly Ile
135                 140                 145                 150

TTC GAC GGC TGC ACC TCT GAG TTC GGC AAG GCT CTC GGC GGT GCC CAG          594
Phe Asp Gly Cys Thr Ser Glu Phe Gly Lys Ala Leu Gly Gly Ala Gln
                155                 160                 165

TAC GGC GGT ATC TCC TCC CGA AGC GAA TGT GAT AGC TAC CCC GAG CTT          642
Tyr Gly Gly Ile Ser Ser Arg Ser Glu Cys Asp Ser Tyr Pro Glu Leu
                170                 175                 180

CTC AAG GAC GGT TGC CAC TGG CGA TTC GAC TGG TTC GAG AAC GCC GAC          690
Leu Lys Asp Gly Cys His Trp Arg Phe Asp Trp Phe Glu Asn Ala Asp
            185                 190                 195

AAC CCT GAC TTC ACC TTT GAG CAG GTT CAG TGC CCC AAG GCT CTC CTC          738
Asn Pro Asp Phe Thr Phe Glu Gln Val Gln Cys Pro Lys Ala Leu Leu
        200                 205                 210

GAC ATC AGT GGA TGC AAG CGT GAT GAC GAC TCC AGC TTC CCT GCC TTC          786
Asp Ile Ser Gly Cys Lys Arg Asp Asp Asp Ser Ser Phe Pro Ala Phe
215                 220                 225                 230

AAG GTT GAT ACC TCG GCC AGC AAG CCC CAG CCC TCC AGC TCC GCT AAG          834
Lys Val Asp Thr Ser Ala Ser Lys Pro Gln Pro Ser Ser Ser Ala Lys
                235                 240                 245

AAG ACC ACC TCC GCT GCT GCT GCC GCT CAG CCC CAG AAG ACC AAG GAT          882
Lys Thr Thr Ser Ala Ala Ala Ala Ala Gln Pro Gln Lys Thr Lys Asp
                250                 255                 260

TCC GCT CCT GTT GTC CAG AAG TCC TCC ACC AAG CCT GCC GCT CAG CCC          930
Ser Ala Pro Val Val Gln Lys Ser Ser Thr Lys Pro Ala Ala Gln Pro
        265                 270                 275

GAG CCT ACT AAG CCC GCC GAC AAG CCC CAG ACC GAC AAG CCT GTC GCC          978
Glu Pro Thr Lys Pro Ala Asp Lys Pro Gln Thr Asp Lys Pro Val Ala
    280                 285                 290

ACC AAG CCT GCT GCT ACC AAG CCC GTC CAA CCT GTC AAC AAG CCC AAG          1026
Thr Lys Pro Ala Ala Thr Lys Pro Val Gln Pro Val Asn Lys Pro Lys
295                 300                 305                 310

ACA ACC CAG AAG GTC CGT GGA ACC AAA ACC CGA GGA AGC TGC CCG GCC          1074
Thr Thr Gln Lys Val Arg Gly Thr Lys Thr Arg Gly Ser Cys Pro Ala
        315                 320                 325
```

20

```
AAG ACT GAC GCT ACC GCC AAG GCC TCC GTT GTC CCT GCT TAT TAC CAG       1122
Lys Thr Asp Ala Thr Ala Lys Ala Ser Val Val Pro Ala Tyr Tyr Gln
            330             335             340

TGT GGT GGT TCC AAG TCC GCT TAT CCC AAC GGC AAC CTC GCT TGC GCT       1170
Cys Gly Gly Ser Lys Ser Ala Tyr Pro Asn Gly Asn Leu Ala Cys Ala
            345             350             355

ACT GGA AGC AAG TGT GTC AAG CAG AAC GAG TAC TAC TCC CAG TGT GTC       1218
Thr Gly Ser Lys Cys Val Lys Gln Asn Glu Tyr Tyr Ser Gln Cys Val
            360             365             370

CCC AAC TAAATGGTAG ATCCATCGGT TGTGGAAGAG ACTATGCGTC TCAGAAGGGA        1274
Pro Asn
375

TCCTCTCATG AGCAGGCTTG TCATTGTATA GCATGGCATC CTGGACCAAG TGTTCGACCC     1334

TTGTTGTACA TAGTATATCT TCATTGTATA TATTTAGACA CATAGATAGC CTCTTGTCAG     1394

CGACAACTGG CTACAAAAGA CTTGGCAGGC TTGTTCAATA TTGACACAGT TTCCTCCATA     1454

AAAAAAAAAA AAAAAAAAA                                                  1473
```

SEQUENCE DESCRIPTION: SEQ ID NO:4:

```
Met Arg Ser Tyr Thr Leu Leu Ala Leu Ala Gly Pro Leu Ala Val Ser
 1               5                  10                  15

Ala Ala Ser Gly Ser Gly His Ser Thr Arg Tyr Trp Asp Cys Cys Lys
            20              25                  30

Pro Ser Cys Ser Trp Ser Gly Lys Ala Ala Val Asn Ala Pro Ala Leu
        35              40                  45

Thr Cys Asp Lys Asn Asp Asn Pro Ile Ser Asn Thr Asn Ala Val Asn
    50                  55                  60

Gly Cys Glu Gly Gly Gly Ser Ala Tyr Ala Cys Thr Asn Tyr Ser Pro
65                  70                  75                  80

Trp Ala Val Asn Asp Glu Leu Ala Tyr Gly Phe Ala Ala Thr Lys Ile
            85                  90                  95

Ser Gly Gly Ser Glu Ala Ser Trp Cys Cys Ala Cys Tyr Ala Leu Thr
            100             105             110

Phe Thr Thr Gly Pro Val Lys Gly Lys Lys Met Ile Val Gln Ser Thr
    115             120             125

Asn Thr Gly Gly Asp Leu Gly Asp Asn His Phe Asp Leu Met Met Pro
    130             135             140

Gly Gly Gly Val Gly Ile Phe Asp Gly Cys Thr Ser Glu Phe Gly Lys
145             150             155             160

Ala Leu Gly Gly Ala Gln Tyr Gly Gly Ile Ser Ser Arg Ser Glu Cys
            165             170             175

Asp Ser Tyr Pro Glu Leu Leu Lys Asp Gly Cys His Trp Arg Phe Asp
            180             185             190

Trp Phe Glu Asn Ala Asp Asn Pro Asp Phe Thr Phe Glu Gln Val Gln
    195             200             205

Cys Pro Lys Ala Leu Leu Asp Ile Ser Gly Cys Lys Arg Asp Asp Asp
    210             215             220

Ser Ser Phe Pro Ala Phe Lys Val Asp Thr Ser Ala Ser Lys Pro Gln
225             230             235             240
```

```
Pro Ser Ser Ser Ala Lys Lys Thr Thr Ser Ala Ala Ala Ala Ala Gln
            245             250                 255

Pro Gln Lys Thr Lys Asp Ser Ala Pro Val Val Gln Lys Ser Ser Thr
            260             265                 270

Lys Pro Ala Ala Gln Pro Glu Pro Thr Lys Pro Ala Asp Lys Pro Gln
            275             280                 285

Thr Asp Lys Pro Val Ala Thr Lys Pro Ala Ala Thr Lys Pro Val Gln
    290                 295                 300

Pro Val Asn Lys Pro Lys Thr Thr Gln Lys Val Arg Gly Thr Lys Thr
305                 310                 315                 320

Arg Gly Ser Cys Pro Ala Lys Thr Asp Ala Thr Ala Lys Ala Ser Val
            325             330                 335

Val Pro Ala Tyr Tyr Gln Cys Gly Gly Ser Lys Ser Ala Tyr Pro Asn
            340             345                 350

Gly Asn Leu Ala Cys Ala Thr Gly Ser Lys Cys Val Lys Gln Asn Glu
            355             360                 365

Tyr Tyr Ser Gln Cys Val Pro Asn
370                 375
```

## Claims

1. A dye transfer inhibiting composition comprising

   - an enzyme exhibiting peroxidase activity.
   - a hydrogen peroxide or a hydrogen peroxide precursor, or an enzymatic system capable of generating hydrogen peroxide.
   - an additional oxidizable substrate, and
   - a cellulase preparation, characterized in that the cellulase preparation provides at least 10% removal of immobilized radioactive labelled carboxymethyl cellulose according to the C14CMC method at 25 x 10$^{-6}$% by weight of the cellulase protein in the test solution.

2. A dye transfer inhibiting composition according to claim 1 wherein the cellulase preparation consists essentially of a homogeneous endoglucanase component which is immunoreactive with an antibody raised against a highly purified about 43 kD endoglucanase [measured by SDS PAGE] derived from Humicola insolens, DSM 1800, or which is homologous to said 43kD endoglucanase [measured by SDS - PAGE].

3. A dye transfer inhibiting composition according to claim 2 wherein the endoglucenase component of said cellulase preparation has an isoelectric paint of about 5.1.

4. A dye transfer inhibiting composition according to claims 2 or 3 wherein said endoglucanase component is producible by a method comprising cultivating a host sell transformed with a recombinant DNA vector carrying a DNA sequence encoding said endoglucanase component or a precursor of said endoglucanase component, as well as DNA sequences encoding functions permitting the expression of the DNA sequence encoding the endoglucanase component or a precursor thereof, in a culture medium under conditions permitting the expression of the endoglucanase component or precursor thereof and recovering the endoglucanase component form the culture.

**5.** A dye transfer inhibiting composition according to claim 1 wherein the cellulase preparation consists essentially of an homogenous endoglucanase component having the amino acid sequence shown in the appended sequence listing ID#2, or is a homologue thereof exhibiting endoglucanase activity.

**6.** A dye transfer inhibiting composition according to claim 3 wherein said endoglucanase is producible by a species of Humicola, e.g. Humicola insolens.

**7.** A dye transfer inhibiting composition according to claim 1 characterized in that the cellulase preparation consists essentially of an homogeneous endoglucanase component having the amino acid sequence shown in the appended sequence listing ID#4, or is a homologue thereof exhibiting endoglucanase activity.

**8.** A dye transfer inhibiting composition according to claim 7 wherein said endoglucanase enzyme is producible by a species of Fusarium, e.g. Fusarium oxysporum.

**9.** A dye transfer inhibiting composition according to any of the claims 5 to 8 wherein said enzyme is produced by a DNA construct comprising a DNA sequence encoding the enzyme.

**10.** A dye transfer inhibiting composition according to claim 9 wherein the DNA sequence is as shown in the appended sequence listings ID#1 or ID#3.

**11.** A dye transfer inhibiting composition according to any of the claims 5 to 10 wherein said enzyme is producible by a host cell being a strain of a fungus such as Tricloderuca or Aspergillus, preferably Aspergillus oryzae or Aspergillus niger, or a yeast cell belonging to a strain of Hansenula or Saccharomyces, e.g. a strain of Saccachomyces cerevisae.

**12.** A dye transfer inhibiting composition according to any of the claims 5 to 10 wherein said enzyme is producible by a host cell being a strain of a bacterium, e.g. Bacillus, Streptomyces or E. coli.

**13.** A dye transfer inhibiting composition according to claims 1 to 12, wherein said enzyme exhibiting peroxidase activity is derived from a strain of Coprinus or B. purilus.

**14.** A dye transfer inhibiting composition according to claims 1 to 13, wherein the hydrogen peroxide precursor is a perborate or percarbonate.

**15.** A dye transfer inhibiting composition according to claim 14 wherein the level of said perborate is from 1 $\mu$M - 10mM of the wash solution.

**16.** A dye transfer inhibiting composition according to claims 1-15, wherein said additional oxidizable substrate is selected from a metal ion, a halide ion or an organic compound such as a phenol, e.g. p-hydroxycinnamic acid or 2,4-dichlorophenol, or a phenol sulfonate.

**17.** A dye transfer inhibiting composition according to any of claims 1-16, wherein said enzymatic system capable of generating hydrogen peroxide is an oxidase selected from the group consisting of glucose oxidase, urate oxidase, galactose oxidase, alcohol oxidases, amine oxidases, amino acid oxidase and cholesterol oxidase.

**18.** A dye transfer inhibiting composition according to any of the preceding claims, which is a detergent additive, in the form of a non-dusting granulate, a liquid, in particular a stabilized liquid, or a protected enzyme system.

**19.** A detergent composition which comprises a detergent additive according to claim 18.

**20.** A detergent composition according to claim 19 which is in granular form, compact granular form or liquid form.

**21.** A process for inhibiting the transfer of a textile dye from a dyed fabric to another fabric when said fabrics am washed and/or rinsed together in a wash liquor, comprising contacting said fabrics with a composition according to claims 1 to 20 above, the peroxidase being used at levels of from 0.01 to 100 mg/liter of wash solution, the level of hydrogen peroxide being from 0.001 - 5 mM of the wash solution, and the absolute level of additional oxidizable substrate being from 1 $\mu$M to 1 mM, and the cellulase being added at levels of from 0.005 to 40 mg enzyme protein/liter of wash solution.

**Patentansprüche**

1. Die Farbstoffübertragung inhibierende Zusammensetzung, umfassend:

   - ein Peroxidaseakivität zeigendes Enzym,
   - Wasserstoffperoxid oder einen Wasserstoffperoxidvorläufer oder ein Enzymsystem, das Wasserstoffperoxid erzeugen kann,
   - ein zusätzliches oxidierbares Substrat, und
   - eine Cellulasezubereitung, dadurch gekennzeichnet, daß die Cellulasezubereitung eine mindestens 10%ige Entfernung von immobilisierter, radioaktiv markierter Carboxymethylcellulose gemäß dem C14CMC-Verfahren bei $25 \times 10^6$%, bezogen auf das Gewicht des Cellulaseproteins in der Testlösung, vorsieht.

2. Die Farbstofübertragung inhibierende Zusammensetzung nach Anspruch 1, wobei die Cellulasezubereitung im wesentlichen aus einer homogenen Endoglucanasekomponente besteht, die mit einem Antikörper inmunreaktiv ist, der gegen eine hochreine, etwa 43 kD-Endoglucanase [bestimmt durch eine SDS-PAGE] erzeugt wurde, die von Humicola insolens, DSM 1800, stammt oder die zu der 43kD-Endoglucanase [bestimmt durch eine SDS-PAGE] homolog ist.

3. Die Farbstoffübertragung inhibierende Zusammensetzung nach Anspruch 2, wobei die Endoglucanasekomponente der Cellulasezubereitung einen isoelektrischen Punkt von etwa 5,1 aufweist.

4. Die Farbstoffübertragung inhibierende Zusammensetzung nach Anspruch 2 oder 3, wobei die Endoglucanasekomponente durch ein Verfahren hergestellt werden kann, umfassend die Züchtung einer Wirtszelle, die mit einem rekombinanten DNA-Vektor transformiert wurde, enthaltend eine DNA-Sequenz, welche die Endoglucanasekomponente oder einen Vorläufer der Endoglucanasekomponente codiert, sowie DNA-Sequenzen, die Funktionen codieren, welche die Expression der DNA-Sequenz zulassen, die die Endoglucanasekomponente oder einen Vorläufer hiervon codiert, in einem Kulturmedium unter Bedingungen, welche die Expression der Endoglucanasekomponente oder eines Vorläufers hiervon und die Gewinnung der Endoglucanasekomponente aus der Kultur erlaubt.

5. Die Farbstoffübertragung inhibierende Zusammensetzung nach Anspruch 1, wobei die Cellulasezubereitung im wesentlichen aus einer homogenen Endoglucanasekomponente mit der in der beigefügten Sequenzliste ID#2 gezeigten Aminosäuresequenz besteht oder ein Homolog hiervon ist, das Endoglucanaseaktivität zeigt.

6. Die Farbstoffübertragung inhibierende Zusammensetzung nach Anspruch 3, wobei die Endoglucanase durch eine Art von Humicola, z.B. Humicola insolens, hergestellt werden kann.

7. Die Farbstoffübertragung inhibierende Zusammensetzung nach Anspruch 1, dadurch gekennzeichnet, daß die Cellulasezubereitung im wesentlichen aus einer homogenen Endoglucanasekomponente mit der in der beigefügten Sequenzliste ID#4 gezeigten Aminosäuresequenz besteht oder ein Homolog hiervon ist, das Endoglucanaseaktivität zeigt.

8. Die Farbstoffübertragung inhibierende Zusammensetzung nach Anspruch 7, wobei das Endoglucanaseenzym durch eine Art von Fusarium, z.B. Fusarium oxysporum, hergestellt werden kann.

9. Die Farbstoftübertragung inhibierende Zusammensetzung nach einem der Ansprüche 5 bis 8, wobei das Enzym durch ein DNA-Konstrukt hergestellt wird, das eine das Enzym codierende DNA-Sequenz umfaßt.

10. Die Farbstoffübertragung inhibierende Zusammensetzung nach Anspruch 9, wobei die DNA-Sequenz ist, wie in den beigefügten Sequenzlisten ID#1 oder ID#3 gezeigt.

11. Die Farbstoffübertragung inhibierende Zusammensetzung nach einem der Ansprüche 5 bis 10, wobei das Enzym durch eine Wirtszelle, die ein Stamm eines Pilzes wie Tricloderuca oder Aspergillus, vorzugsweise Aspergillus oryzae oder Aspergillus niger, ist, oder eine Hefezelle, die zu einem Stamm von Hansenula oder Saccharomyces, z.B. einem Stamm von Saccharomyces cerevisae, gehört, hergestellt werden kann.

12. Die Farbstoffübertragung inhibierende Zusammensetzung nach einem der Ansprüche 5 bis 10, wobei das Enzym durch eine Wirtszelle hergestellt werden kann, die ein Stamm eines Bakteriums, z.B. Bacillus, Streptomyces oder E. coli, ist.

**13.** Die Farbstoffübertragung inhibierende Zusammensetzung nach Anspruch 1 bis 12, wobei das Peroxidaseaktivität zeigende Enzym von einem Stamm von Coprinus oder B. purilus stammt.

**14.** Die Farbstoffübertragung inhibierende Zusammensetzung nach Anspruch 1 bis 13, wobei der Wasserstoffperoxidvorläufer ein Perborat oder Percarbonat ist.

**15.** Die Farbstoffübertragung inhibierende Zusammensetzung nach Anspruch 14, wobei der Anteil des Perborats 1 μM-10 mM der Waschlösung beträgt.

**16.** Die Farbstoffübertragung inhibierende Zusammensetzung nach Anspruch 1 bis 15, wobei das zusätzliche oxidierbare Substrat ausgewählt ist aus einem Metallion, einem Halogenidion oder einer organischen Verbindung, wie ein Phenol, z.B. p-Hydroxyzimtsäure oder 2,4-Dichlorpenol, oder ein Phenolsulfonat.

**17.** Die Farbstoffübertragung inhibierende Zusammensetzung nach einem der Ansprüche 1 bis 16, wobei das Enzymsystem, das Wasserstoffperoxid erzeugen kann, eine Oxidase ist, gewählt aus der Gruppe, bestehend aus Glucoseoxidase, Uratoxidase, Galactoseoxidase, Alkoholoxidasen, Aminoxidasen, Aminosäureoxidase und Cholesterinoxidase.

**18.** Die Farbstoffübertragung inhibierende Zusammensetzung nach einem der vorhergehenden Ansprüche, die ein Waschmittelzusatz ist, in Form eines nicht-pulverförmigen Granulats, einer Flüssigkeit, insbesondere einer stabilisierten Flüssigkeit, oder eines geschützten Enzymsystems.

**19.** Waschmittelzusammensetzung, die einen Waschmittelzusatz nach Anspruch 18 umfaßt.

**20.** Waschmittelzusammensetzung nach Anspruch 19, die in granulärer Form, kompakter granulärer Form oder flüssiger Form vorliegt.

**21.** Verfahren zur Inhibierung der Übertragung eines Textilfarbstoffs von einem gefärbten Textil auf andere Textilien, wenn die Textilien zusammen in einer Waschflotte gewaschen und/oder gespült werden, umfassend das Inkontaktbringen der Textilien mit einer Zusammensetzung nach einem der vorstehenden Ansprüche 1 bis 20, wobei die Peroxidase in Anteilen von 0,01 bis 100 mg/Liter Waschlösung verwendet wird, der Anteil an Wasserstoffperoxid 0,001-5 mM der Waschlösung ausmacht und der absolute Anteil des zusätzlichen oxidierbaren Substrats 1 μM bis 1 mM beträgt, und die Cellulase in Anteilen von 0,005 bis 40 mg Enzymprotein/Liter Waschlösung zugesetzt wird.

## Revendications

**1.** Composition inhibant le transfert des colorants, comprenant :

- une enzyme présentant une activité de peroxydase,
- du peroxyde d'hydrogène ou un précurseur de peroxyde d'hydrogène, ou encore un système enzymatique capable de générer du peroxyde d'hydrogène,
- un substrat oxydable additionnel, et
- une préparation de cellulase,
  caractérisée en ce que la préparation de cellulase réalise au moins 10 % d'enlèvement de carboxyméthylcellulose marquée par un composé radioactif immobilisé, conformément au procédé à la $^{14}$C-CMC, pour 25 x 10$^{-6}$ % en poids de la protéine cellulase dans la solution testée.

**2.** Composition inhibant le transfert des colorants selon la revendication 1, dans laquelle la préparation de cellulase est constituée principalement d'un composant endoglucanase homogène qui est immunoréactif avec un anticorps dirigé contre une endoglucanase hautement purifiée d'environ 43 kD (mesuré par SDS-PAGE) dérivant de <u>Humicola insolens</u> DSM 1800 ou qui est homologue de ladite endoglucanase de 43 kD (mesuré par SDS-PAGE).

**3.** Composition inhibant le transfert des colorants selon la revendication 2, dans laquelle le composant endoglucanase de ladite préparation de cellulase a un point isoélectrique d'environ 5,1.

**4.** Composition inhibant le transfert des colorants selon la revendication 2 ou 3, dans laquelle ledit composant endoglucanase est productible par un procédé consistant à cultiver une cellule hôte transformée avec un vecteur d'ADN recombinant portant une séquence d'ADN codant ledit composant endoglucanase ou un précurseur dudit composant endoglucanase, ainsi que des séquences d'ADN codant des fonctions permettant l'expression de la séquence d'ADN codant le composant endoglucanase, ou un précurseur de celui-ci, dans un milieu de culture dans des conditions permettant l'expression du composant endoglucanase ou de son précurseur, et à récupérer le composant endoglucanase à partir de la culture.

**5.** Composition inhibant le transfert des colorants selon la revendication 1, dans laquelle la préparation de cellulase est constituée principalement d'un composant endoglucanase homogène ayant la séquence d'acides aminés présentée dans la ID#2 de la liste de séquences annexée, ou est un homologue de celui-ci présentant une activité d'endoglucanase.

**6.** Composition inhibant le transfert des colorants selon la revendication 3, dans laquelle ladite endoglucanase est productible par une espèce de Humicola, par exemple Humicola insolens.

**7.** Composition inhibant le transfert des colorants selon la revendication 1, caractérisée en ce que la préparation de cellulase est constituée principalement d'un composant endoglucanase homogène ayant la séquence d'acides aminés présentée dans la ID#4 de la liste de séquences annexée, ou est un homologue de celui-ci présentant une activité d'endoglucanase.

**8.** Composition inhibant le transfert des colorants selon la revendication 7, dans laquelle ladite enzyme endoglucanase est productible par une espèce de Fusarium, par exemple Fusarium oxysporum.

**9.** Composition inhibant le transfert des colorants selon l'une quelconque des revendications 5 à 8, dans laquelle ladite enzyme est produite par un ADN hybride comprenant une séquence d'ADN codant l'enzyme.

**10.** Composition inhibant le transfert des colorants selon la revendication 9, dans laquelle la séquence d'ADN est telle que présentée dans la ID#1 ou la ID#3 de la liste de séquences annexée.

**11.** Composition inhibant le transfert des colorants selon l'une quelconque des revendications 5 à 10, dans laquelle ladite enzyme est productible par une cellule hôte qui est une souche d'un champignon tel que Tricloderuca ou Aspergillus, de préférence Aspergillus oryzae ou Aspergillus niger, ou une cellule de levure appartenant à une souche de Hansenula ou Saccharomyces, par exemple une souche de Saccharomyces cerevisiae.

**12.** Composition inhibant le transfert des colorants selon l'une quelconque des revendications 5 à 10, dans laquelle ladite enzyme est productible par une cellule hôte qui est une souche d'une bactérie, par exemple Bacillus, Streptomyces ou E. coli.

**13.** Composition inhibant le transfert des colorants selon les revendications 1 à 12, dans laquelle ladite enzyme présentant une activité de peroxydase dérive d'une souche de Coprinus ou B. purilus.

**14.** Composition inhibant le transfert des colorants selon les revendications 1 à 13, dans laquelle le précurseur de peroxyde d'hydrogène est un perborate ou un percarbonate.

**15.** Composition inhibant le transfert des colorants selon la revendication 14, dans laquelle la quantité dudit perborate est de 1 $\mu$M à 10 mM de la solution de lavage.

**16.** Composition inhibant le transfert des colorants selon les revendications 1 à 15, dans laquelle ledit substrat oxydable additionnel est choisi parmi un ion métallique, un ion halogénure ou un composé organique tel qu'un phénol, par exemple l'acide p-hydroxycinnamique ou le 2,4-dichlorophénol, ou un phénolsulfonate.

**17.** Composition inhibant le transfert des colorants selon l'une quelconque des revendications 1 à 16, dans laquelle ledit système enzymatique capable de générer du peroxyde d'hydrogène est une oxydase choisie dans le groupe constitué par la glucose oxydase, l'urate oxydase, la galactose oxydase, les alcool oxydases, les amine oxydases, les acide aminé oxydases et la cholestérol oxydase.

**18.** Composition inhibant le transfert des colorants selon l'une quelconque des revendications précédentes, qui est un additif détergent, sous la forme d'un produit de granulation non poudreux, d'un liquide, en particulier d'un liquide stabilisé, ou d'un système enzymatique protégé.

**19.** Composition détergente qui comprend un additif détergent selon la revendication 18.

**20.** Composition détergente selon la revendication 19, qui est sous forme granulaire, sous forme granulaire compacte ou sous forme liquide.

**21.** Procédé pour inhiber le transfert d'un colorant textile d'un tissu teint à un autre tissu quand lesdits tissus sont lavés et/ou rincés ensemble dans une liqueur de lavage, qui consiste à mettre lesdits tissus en contact avec une composition selon les revendications 1 à 20 ci-dessus, la peroxydase étant utilisée en des quantités de 0,01 à 100 mg par litre de solution de lavage, la quantité de peroxyde d'hydrogène étant de 0,001 à 5 mM de la solution de lavage, et la quantité absolue de substrat oxydable additionnel étant de 1 $\mu$M à 1 mM, et la cellulase étant ajoutée en des quantités de 0,005 à 40 mg de protéine d'enzyme par litre de solution de lavage.